# EUROPÄISCHE PATENTANMELDUNG

(11) **EP 0 528 202 A1**
(43) Veröffentlichungstag der Anmeldung: **24.02.1993**
(21) Anmeldenummer: 92112585.2
(22) Anmeldetag: 23.07.1992
(51) Int. Cl.: A01M 1/20, A61L 9/12

(54) **Verdunstungsvorrichtung, insbesondere für flüchtige Wirkstoffe wie Insektizide, Duftstoffe oder dergleichen**

(30) Priorität: 21.08.1991 DE 4127692
(71) Anmelder: Globol GmbH, D-86633 Neuburg (DE)
(72) Erfinder: Hautmann, Horst, Ing. Chem., W-8858 Neuburg/Donau (DE)
(74) Vertreter: Reinhard - Skuhra - Weise & Partner

(57) **Zusammenfassung**

Eine Verdunstungsvorrichtung, insbesondere für flüchtige Wirkstoffe wie Insektizide, Duftstoffe oder dergleichen weist ein Gehäuse (1,2) mit Durchbrechungen (9) auf, das aus wenigstens zwei um eine Schwenkachse beweglichen Gehäuseteilen (1,2) besteht. In dem Gehäuse ist mindestens ein Aufnahmeabschnitt (10) für einen Behälter für Wirkstoffe, und mindestens eine dem Behälter (54) zugeordneten Öffnereinheit (40,41) vorgesehen. Dabei ist eine Einrichtung (14,55) zur Anzeige des Betriebszustandes vorgesehen.

## Beschreibung

Die Erfindung betrifft eine Verdunstungsvorrichtung gemäß dem Oberbegriff des Anspruchs 1.

Eine Verdunstungsvorrichtung gemäß dem Oberbegriff des Patentanspruchs 1 ist aus der EP 094 499, Fig. 7 bis 9, bekannt. Ein Gehäuseteil enthält eine Öffnereinheit, die mit einem perforierbaren Behälter zusammenwirkt, der in einem anderen Gehäuseteil aufgenommen ist. Die Perforierung des Behälters erfolgt durch Verschwenken des ihn aufnehmenden Gehäuseteils in Richtung auf die Öffnereinheit. Bei dieser Verdunstungsvorrichtung ist der Füllstand des Behälters von außen nicht erkennbar. Der Benutzer ist gehalten, zur Überprüfung des Füllstandes entweder den Ausdunstungsgrad der Verdunstungsvorrichtung zu überprüfen oder die Gehäuseteile zur Inspizierung des Behälters zu öffnen.

Bei einer anderen Ausbildung einer Verdunstungsvorrichtung entsprechend Fig. 1 der EP 094 499 sind die die Wirkstoffe aufnehmenden Behälter so angeordnet, daß sie von der Gehäuseaußenseite her sichtbar sind und im Falle von transparenten Behältern für Wirkstoffe deren Füllstand von außen erkennbar ist.
Bei der beschriebenen Verdunstungsvorrichtung ist vorgesehen, die Perforierung der die Wirkstoffe aufnehmenden Behälter zum Zwecke des Austretens der Wirkstoffe derart vorzunehmen, daß vergleichbar kleine Öffnungen in die perforierbare Fläche eingebracht werden und die Wirkstoffe langsam aus jedem Behälter ausfließen.
Derartige Verdunstungsvorrichtungen sind somit nicht geeignet, eine vergleichbar große Quantität von Wirkstoffen verdunsten zu lassen, um zum Beispiel starke Geruchsprobleme zu bekämpfen.

Der Erfindung liegt die Aufgabe zugrunde, eine Verdunstungsvorrichtung der eingangs genannten Art so zu verbessern, daß sie einen hohen Verdunstungswirkungsgrad gewährleistet und bei welcher der Betriebszustand des Geräts leicht erkennbar ist.

Diese Aufgabe wird erfindungsgemäß durch eine Einrichtung zur Anzeige des Betriebszustandes gelöst.

Weitere Ausgestaltungen der Verdunstungsvorrichtung ergeben sich aus den Unteransprüchen.

Die Erfindung schafft eine Verdunstungsvorrichtung, bei welcher der in wenigstens einem Behälter befindliche Wirkstoff nach Betätigung der Verdunstungsvorrichtung zur Ermöglichung einer intensiven Wirkstoffverdunstung praktisch vollständig ausfließt und bei der eine Einrichtung zur Überprüfung des Betriebszustandes vorgesehen ist.

Gemäß einer bevorzugten Ausführungsform der Verdunstungsvorrichtung läßt sich ein Trägerelement in dem Gehäuse anordnen, in welches der oder die Behälter mit Wirkstoff und die Einrichtung zur Anzeige des Betriebszustandes eingesetzt sind.

Gemäß einer bevorzugten Ausführungsform der Verdunstungsvorrichtung besteht der den Wirkstoff aufnehmende Behälter und der als Betriebszustandsanzeigeeinrichtung dienende Behälter aus einem vorzugsweise tiefgezogenen Kunststoffteil, das durch eine perforierbare plane Schicht abgeschlossen ist, derart, daß ein umlaufender Rand oder Kragen gebildet wird, mittels welchem jeder Behälter an dem Trägerelement gehaltert werden kann. Jedem Behälter ist eine Öffnereinheit zugeordnet, wobei die Öffnereinheit für denjenigen Behälter, der für die Betriebszustandsanzeige vorgesehen ist, vorzugsweise aus einem Dorn oder dgl. besteht, der nur eine kleine Öffnung in die perforierbare Fläche einbringt. Die dem Behälter mit Wirkstoff zugeordnete Öffnereinheit besteht demgegenüber aus einer vergleichbar großflächigen Öffnereinheit oder zwei oder mehr zueinander beabstandeten Öffnereinheiten besteht, um große oder großflächige Öffnungen erzeugen zu können. Auf diese Weise ist ein schnelles Herausfließen des Wirkstoffes aus dem oder den Behälter(n) nach dessen bzw. deren Öffnung gewährleistet, weil der Wirkstoff aus größeren Austrittsöffnungen herausfließen kann.

Gemäß einer weiteren Ausführungsform ist an der Unterseite der Verdunstungsvorrichtung ein wannenförmiger Behälter zur Aufnahme der aus dem Behälter austretenden bzw. herausfließenden Wirkstofflüssigkeit vorgesehen.

Bei einer bevorzugten Ausführungsform der Verdunstungsvorrichtung ist das Trägerelement aus einem saugfähigen Element, beispielsweise aus Zellstoff, gefertigt, welches in den wannenförmigen Behälter hineinreicht. Damit gelangt der Wirkstoff in intensiven Kontakt mit dem saugfähigen Element und es ist eine Verdunstung über die gesamte Fläche des saugfähigen Elementes gewährleistet, das heißt, es steht eine relativ große Verdunstungsfläche zur Verfügung.

Der oder die Wirkstoffbehälter der Verdunstungsvorrichtung sind von der Außenseite vorzugsweise nicht einsehbar, während der Behälter zur Betriebszustandsanzeige über eine Öffnung von der Gehäuseaußenseite her erkennbar ist. Als Indikatorflüssigkeit wird beispielsweise ein Wasser/Alkohol-Gemisch mit Farbstoffzusatz benutzt. Nach Perforierung dieses Betriebszustandsanzeigebehälters verdunstet dessen Inhalt allmählich, wobei die Zusammensetzung der Indikatorflüssigkeit so gewählt ist, daß sie über einen Zeitraum verdunstet, innerhalb welchem auch die Wirkstofflüssigkeit vollständig verdunstet. Ein leerer Indikatorflüssigkeitsbehälter zeigt also an, daß ein neuer, mit Wirkstoff gefüllter Behälter eingesetzt werden muß.

Der Austausch von Wirkstoffbehälter und Behälter mit Indikatorflüssigkeit läßt sich bei der Verdunstungsvorrichtung dadurch einfach bewerkstelligen, daß das Trägerelement zusammen mit den leeren Behältern aus dem Gehäuse entfernt und durch ein neues Trägerelement mit gefüllten Behältern ersetzt wird.
Nach dem Zusammenklappen der Gehäuseteile werden die Behälter in der gewünschten Weise geöffnet bzw. perforiert und damit in Betrieb gesetzt.

Die Erfindung schafft eine Verdunstungsvorrichtung, die starke Geruchsprobleme wirksam beseitigen kann. Hierbei tritt der Wirkstoff nach der Öffnung der perforierbaren Fläche des den Wirkstoff aufnehmenden Behälters in verhältnismäßig kurzem Zeitraum aus, so daß der betreffende Behälter praktisch sofort bzw. in einem Zuge entleert wird. Der Benutzer kann mit Hilfe einer separaten Anzeigeeinheit feststellen, ob die Verdunstungssvorrichtung wirksam arbeitet oder nicht.

Nachfolgend werden bevorzugte Ausführungsformen der Erfindung anhand der Zeichnungen zur Erläuterung weiterer Merkmale und Vorteile beschrieben.
Es zeigen:
- Fig. 1: eine Perspektivansicht eines ersten Gehäuseteils der Verdunstungsvorrichtung mit einem Aufnahmeabschnitt für den Wirkstoffbehälter und einer Öffnung, durch welche die den Betriebszustand anzeigende Einrichtung von außen sichtbar ist,
- Fig. 2: das dem Gehäuseteil nach Fig. 1 zugeordnete andere Gehäuseteil,
- Fig. 3: eine Aufsicht auf das zwischen den Gehäuseteilen angeordnete Trägerelement,
- Fig. 4a: eine Aufsicht auf die Einrichtung zur Betriebszustandsanzeige,
- Fig. 4b: eine Seitenschnittansicht der Einrichtung nach Fig. 3a,
- Fig. 5: eine im Schnitt gehaltene Perspektivansicht eines Behälters für Wirkstoffe, und
- Fig. 6: eine schematische Darstellung der wesentlichen Teile der Verdunstungsvorrichtung zur Veranschaulichung deren Zuordnung zueinander.

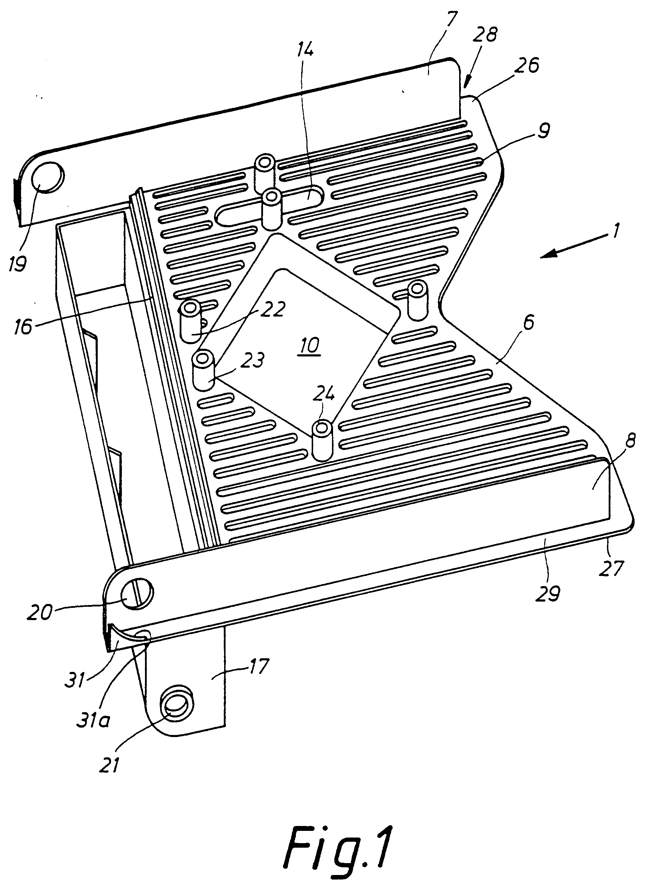

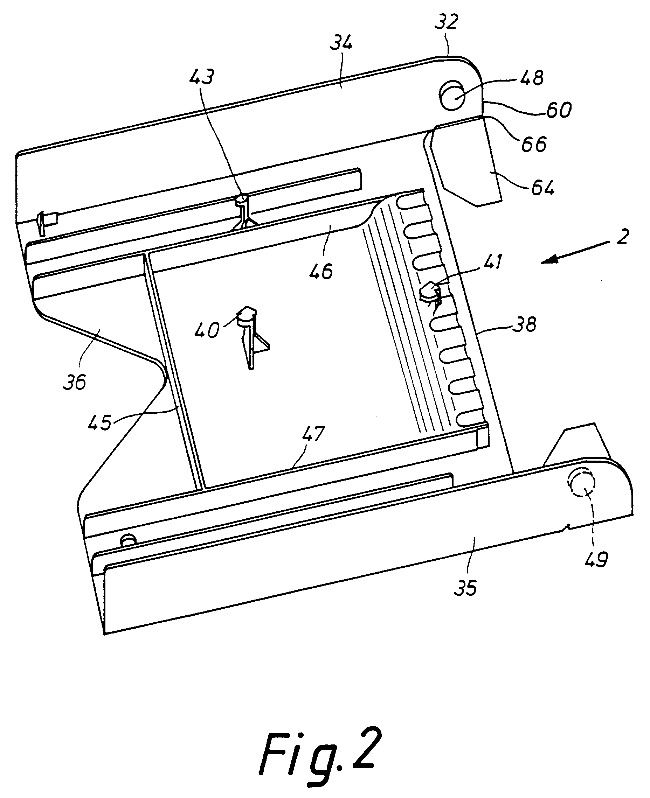

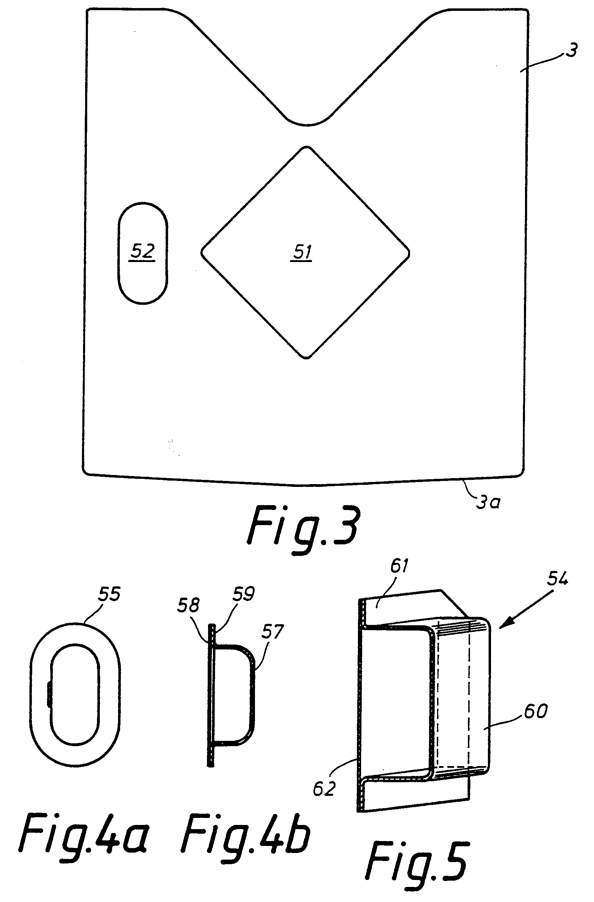

Nachfolgend wird eine bevorzugte Ausführungsform einer Verdunstungsvorrichtung unter Bezugnahme auf die Fig. 1 und 2 erläutert. Das Gehäuse der Verdunstungsvorrichtung besteht aus zwei Gehäuseteilen 1 und 2, die in noch zu beschreibender Weise gegeneinander arretiert werden, um eine Gehäuseeinheit zu ergeben. Zwischen den beiden Gehäuseteilen 1 und 2 wird ein Trägerelement eingesetzt, das in Fig. 3 mit 3 bezeichnet ist.

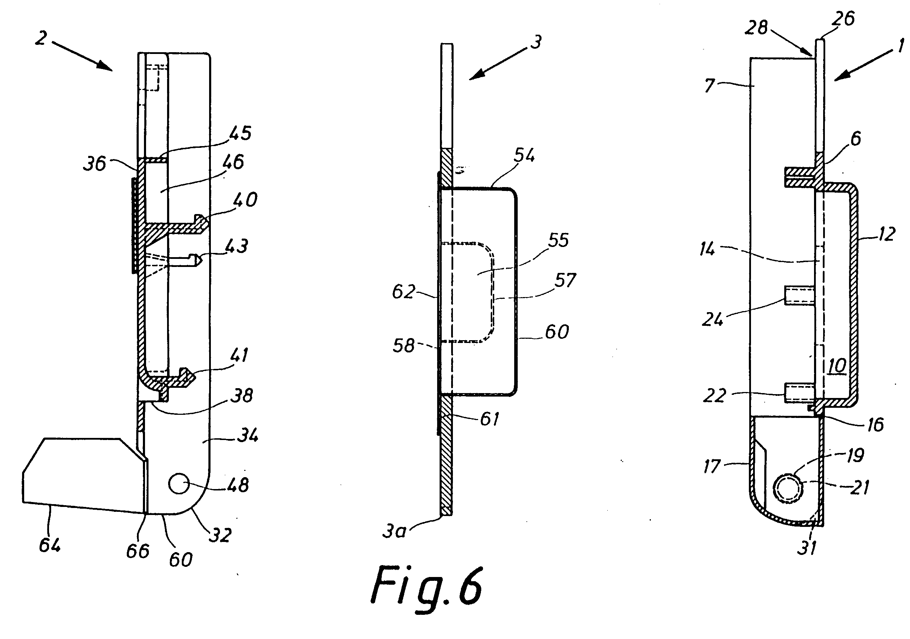

Das Gehäuseteil 1 besteht nach der in Fig. 1 dargestellten Ausführungsform aus einer Gehäusewand 6, an welcher seitlich vorzugsweise vertikal abstrebende Schenkel 7,8 angespritzt sind. Die Gehäusewand 6 weist bei dieser Ausführungsform eine Mehrzahl von Schlitzen oder Durchbrechungen 9 auf, die eine Verdunstung der Wirkstoffe aus dem Gehäuse nach außen heraus ermöglichen. Weiterhin ist in der Gehäusewand 6 ein Aufnahmeabschnitt 10 vorgesehen, der zur Aufnahme eines entsprechend geformten Behälters mit Wirkstofflüssigkeit dient. Der Aufnahmeabschnitt 10 definiert eine nach außen sich erstreckende Vertiefung in der Gehäusewand 6 und ergibt durch den in Fig. 6 mit 12 bezeichneten Boden eine Abdeckung des Behälters für den Wirkstoff. Weiterhin weist die Gehäusewand 6 eine Öffnung 14 auf. Die Öffnung 14 hat vorteilhafterweise schlitzförmige Gestalt, verläuft vertikal und dient dazu, eine hinter dieser Öffnung 14 angeordnete Einrichtung zur Betriebszustandsanzeige des Geräts von außen einsehen zu können.

Bei der dargestellten Ausführungsvariante ist an dem unteren Ende der Gehäusewand 6 ein Filmscharnier 16 ausgebildet. Über dieses Filmscharnier 16 ist ein wannenförmiger Körper 17 gelenkig mit der Gehäusewand 6 verbunden. Gemäß Fig. 1 steht der wannenförmige Körper 17 etwa senkrecht zur Gehäusewand nach unten ab. In diesem Zustand wird das in Fig. 1 gezeigte Teil gespritzt. Nach dem Spritzvorgang, in welchem auch der wannenförmige Körper 17 erzeugt wird, wird dieser Körper 17 um das Filmscharnier 16 in Fig. 1 nach oben im Uhrzeigersinn gedreht, bis er parallel liegt zu den beiden Schenkeln 7,8 (Fig. 6).
Über noch zu beschreibende Mittel wird der Körper 17 gegenüber den Schenkeln 7, 8 fixiert bzw. arretiert.

Bei der in Verbindung mit Fig. 1 beschriebenen Ausführungsform sind beide Schenkel 7,8 an ihrem unteren Ende mit vorzugsweise kreisförmigen Öffnungen 19, 20 versehen, in welche entsprechend ausgebildete, vorzugsweise kreisförmige Vorsprünge 21, die seitlich an dem wannenförmigen Körper 17 abstehen, nach dem Verschwenken des Körpers 17 im Uhrzeigersinn um das Filmscharnier 16 aus der in Fig. 1 gezeigten Position heraus in der in Fig. 6 gezeigten Stellung einrasten. Die Betriebsstellung der Wanne 17 ist aus Fig. 6 erkennbar.

Entsprechend Fig. 1 sind an der Gehäusewand 6 zum Gehäuseinneren abstehende Zapfen 22, 23, 24 usw. oder dergleichen ausgebildet, die dazu dienen, das in das Gehäuseteil 1 eingelegte Trägerelement 3 gegenüber der Gehäusewand 6 um die Höhe der Zapfen 22, 23, 24 usw. beabstandet zu halten.

Das in Fig. 1 gezeigte Gehäuseteil 1 ist derart gestaltet, daß die Schenkel 7,8 gegenüber dem seitlichen Rand 26, 27 der Gehäusewand 6 nach innen versetzt sind. Infolge dessen wird ein zwischen der Kante 26 bzw. 27 und dem Schenkel 7 bzw. 8 befindlicher Rand bzw. Flansch 28, 29 festgelegt, der als Anlagefläche entsprechender Schenkel des zweiten Gehäuseteils 2 dient. Die Schenkel 7,8 stehen von der Gehäusewand 6 in Fig. 1 nach unten über das Filmscharnier 16 über, haben aber insgesamt eine Länge, die der Länge der Gehäusewand 6 zuzüglich der Höhe des wannenförmigen Körpers 17 entspricht.

Am unteren Ende des Flansches oder Randes 28, 29 (Fig. 1) ist eine Einrichtung vorgesehen, die dazu dient, die Schwenkfähigkeit des zweiten Gehäuseteils 2 gegenüber dem Gehäuseteil 1 zu begrenzen. Diese Einrichtung hat vorzugsweise die Form einer spitz zulaufenden Nase 31, die an ihrer in Fig. 1 nach oben weisenden Kantenfläche 31a eine bogenförmige Bahn, das heißt einen Teilkreis, festlegt die weitgehend dem Krümmungsradius der mit dieser Bahn in Kontakt kommenden Kante der Schenkel des Gehäuseteils 2 entspricht. Diese Kante ist in Fig. 2 mit 32 bezeichnet und an jedem Schenkel 34, 35 des Gehäuseteils 2 vorgesehen. Das Gehäuseteil 2 gemäß Fig. 2 weist ebenfalls eine Gehäusewand 36 auf, die im wesentlichen zwischen den beiden Schenkeln 34, 35 liegt. An der Gehäusewand 36 ist eine untere Stirnkante 38 definiert, über welche die Schenkel 34, 35 überstehen. Die beiden Gehäuseteile 1, 2 haben beispielsweise gleiche Höhe und etwa gleiche Breite und bestehen aus Kunststoff:
Das Gehäuseteil 2 nach Fig. 2 ist mit Öffnereinheiten versehen, von denen eine Öffnereinheit durch spitz zulaufende, an der Gehäusewand 36 angespritzte Dorne 40, 41 gebildet ist, während eine zweite Öffnereinheit in Form eines einzelnen Dorns 43 vorgesehen ist. Die Öffnereinheit 40, 41 ist auf der Gehäusewand 36 an einer Position ausgebildet, die etwa der Position des Aufnahmeabschnitts 10 gegenüberliegt, während die Öffnereinheit 43 an einer Stelle von der Gehäusewand 36 absteht, die der Öffnung 14 des Gehäuseteils 1 gegenüberliegt.
Um ein gezieltes Heraustreten und Abfließen der Wirkstofflüssigkeit aus dem noch zu beschreibenden Behälter sicherzustellen, ist die Gehäusewand 36 mit von ihr senkrecht abstehenden Rippen 45,46,47 versehen. Die Rippen 46,47 verlaufen in vertikaler Richtung, die Rippe 45 ist senkrecht zu und oberhalb der Rippen 46, 47 vorgesehen.

Die Spitze bzw. die Form der dornartigen Elemente 40,41,43 ist gegebenenfalls unterschiedlich gewählt, um ein mehr oder weniger starkes Durchdringen der perforierbaren Behälterflächen zu ermöglichen.

An der in Fig. 2 rechten, unteren Seite der Schenkel 34,35 befinden sich nach innen abstrebende, vorzugsweise kreisförmige Zapfen 48,49, deren Abmessungen so gewählt sind, daß sie in Öffnungen eingreifen können, die in den Rastvorsprüngen 21 ausgebildet sind.

Um die beiden Gehäuseteile 1 und 2 gemäß Fig. 1 und 2 zusammenzubauen, ist, wie beschrieben, der wannenförmige Körper 17 aus der in Fig. 1 gezeigten Position im Uhrzeigersinn vorerst um das Filmscharnier 16 zu schwenken, bis die Rastvorsprünge 21 in die Öffnungen 19, 20 eingreifen und der Körper 17 praktisch parallel zu den Schenkeln 7,8, zu liegen kommt. Danach wird das Gehäuseteil 2 derart in das Gehäuseteil 1 eingesetzt, daß die Zapfen 48,49 in die in den Rastvorsprüngen 21 ausgebildeten, in Fig. 1 nur angedeuteten Öffnungen bzw. Vertiefungen der Rastvorsprünge 21 von außen eingreifen. Die Schenkel 34, 35 sind gegenüber den Schenkein 7,8 seitlich nach außen versetzt und übergreifen die Schenkel 7, 8 wenn die beiden Gehäuseteile 1,2 um ihre gemeinsame Schwenkachse aufeinander zu bewegt werden. Die Schwenkachse ist durch die Teile 19,20,48,49 definiert. Nach dem Zusammenbau der beiden Gehäuseteile 1 und 2 läßt sich das Trägerelement 3 einsetzen, wie dies nachfolgend erläutert wird.

Nahe der oberen Stirnkante beispielsweise des Gehäuseteils 2 sind Rastelemente 50a, 50b vorgesehen, die mit den Schenkeln 7, 8 des Gehäuseteils 1 in Eingriff gebracht werden und dadurch die beiden Gehäuseteile 1,2 in paralleler Lage zusammenhalten.

Das Trägerelement 3 ist für die in Fig. 1 und 2 gezeigte Ausführungsform derart ausgebildet, daß es im wesentlichen zwei Öffnungen bzw. Durchbrechungen 51,52 enthält. Das weitgehend rechteckige Trägerelement 3 weist die eine Öffnung 51 an einer Position auf, die nach dem Einsetzen des Trägerelements 3 weitgehend deckungsgleich angeordnet ist mit dem Aufnahmeabschnitt 10, während die Öffnung 52 an einer Position vorgesehen ist, die weitgehend der Öffnung 14 des Gehäuseteils 1 gegenüberliegt. In entsprechender Zuordnung sind die Öffnereinheiten 40, 41 bzw. 43 am Gehäuseteil 2 ausgebildet, d.h. die Öffnerheit 40,41 in Form der Dorne 40,41 ist auf den Bereich der Öffnung 51 des Trägerelementes 3 ausgerichtet, während der Dorn 43 in Richtung auf die Öffnung 42 des in das Gehäuse eingelegten Trägerelements 3 weist. Das Trägerelement 3 (Fig. 3) dient zur Aufnahme von wenigstens zwei Behältern, die unter Bezugnahme auf Fig. 4 und 5 erläutert werden.

In die Öffnung 51 des Trägerelements 3 wird ein Behälter 54 gemäß Fig. 4 und in die Öffnung 52 ein Behälter 55 entsprechend Fig. 4a und 4b eingesetzt. Zuerst wird der Behälter 55 beschrieben. Der Behälter 55 dient als Einrichtung zur Betriebszustandsanzeige, d.h er hat jede beliebige, vorzugsweise längliche Form und besteht aus einem tiefgezogenen Kunststoffteil oder einem etwa napfförmigen Teil 57 aus Kunststoff, das transparente Eigenschaft hat. Die Öffnung dieses Teiles 57 wird nach Befüllung mit einer Flüssigkeit durch eine perforierbare Folie 58 oder dgl. abgeschlossen. Als Flüssigkeit dient vorzugsweise ein Wasser/Alkohol-Gemisch mit Farbstoff, das nach Perforierung der Folie oder Schicht 58 allmählich aus dem Behälter 55 langsam austritt bzw. verdunstet. Die Öffnung 52 im Trägerelement 3 entspricht nahezu exakt der Form des Behälterteils 57 und hat vorzugsweise die Form eines vertikalen Schlitztes. Wie Fig. 4b zeigt, ist an dem Behälterteil 57 ein Flansch 59 ausgebildet. Der Flansch 59 dient dazu, die perforierbare Folie 58 dicht zu lagern. Der Behälter 54 gemäß Fig. 5 ist praktisch ebenso aufgebaut wie der Behälter 55, besitzt jedoch vorzugsweise einen rechteckigen oder quadratischen Behälterkörper 60, an dem nach außen abstrebend ein umlaufender Flansch 61 angeformt ist, der zur Befestigung einer perforierbaren Folie 62 dient. Die Öffnung 51 im Trägerelement 3 entspricht der Form des Behälterkörpers 60. Somit lassen sich die Behälter 55 und 54 in das Trägerteil 3 einsetzen, wobei die jeweiligen Flanschabschnitte 59 und 61 auf der einen Seite des Trägerelementes 3 mit dessen einer Außenfläche in Berührung stehen und der Behälterkörper 57 bzw. 60 auf der anderen Seite des Trägerelements 3 aus der zugehörigen Öffnung 52 bzw. 51 vorsteht. Auf diese Weise werden die beiden Behälter 54, 55 an bzw. im Trägerelement 3 gehaltert und durch das Trägerelement 3 gegenüber den Öffnereinheiten einerseits und der Öffnung 52 bzw. 51 andererseits positioniert.

Fig. 6 zeigt eine im Schnitt gehaltene Seitenansicht der drei wesentlichen Teile 1, 2 und 3 der Verdunstungsvorrichtung in auseinandergezogenem Zustand. Aus Fig. 6 ist ersichtlich, daß das Trägerelement 3 eine Höhe hat, die im wesentlichen der Höhe der Gehäuseteile 1 und 2 entspricht. Zusammen mit den Behältern 54, 55 wird das Trägerelement 3 derart in das Gehäuseteil 1 eingesetzt, daß die untere Kante 3a des Trägerelementes 3 innerhalb des wannenförmigen Körpers 17 zu liegen kommt.
Nach Zusammenklappen der Gehäuseteile 1 und 2 wird automatisch jeder Behälter 54, bzw. 55 durch die zugehörigen Dorne 40, 41 bzw. 43 geöffnet bzw. perforiert, infolge dessen die Flüssigkeit aus dem Behälter 54 nach unten in die Wanne 17 fließen kann. Die Wirkstofflüsigkeit wird vom Trägerelement 3, das vorzugsweise aus Zellstoff besteht, aufgesaugt. Auf diese Weise wird durch das Trägerelement 3 eine Verdunstungsfläche definiert, die der gesamten Fläche des Trägerelementes 3 entspricht.

Aus Fig. 6 ist ersichtlich, daß nach dem Zusammenbau der Gehäuseteile 1 und 2 und deren Drehung um die durch die Gelenkzapfen 48 bestimmte Achse des Gehäuseteils 2 aufeinander zu die Dorne 40,41 und 43 in Richtung auf die Behälter 54, 55 bewegt werden. Im Verlaufe der Schwenkbewegung zum Beispiel, des Gehäuseteils 2 um die durch die Gelenkzapfen 48 definierte Achse in Richtung auf das Gehäuseteil 1, durchdringen die Dorne 40 bis 43 die zugeordneten perforierbaren Folien 62, 58.
Damit ist einerseits eine Entleerung des Behälters 54 sichergestellt und andererseits eine Perforierung der Folie 58 in vorgegebenem Maße. Die Verdunstungsvorrichtung wird durch das Zusammenklappen der beiden Gehäuseteile 1 und 2 automatisch in Betrieb gesetzt.

Die Größe des Behälters 55 und Art des darin enthaltenen Gemisches werden derart gewählt, daß nach einem Zeitraum der Inhalt des Behälters verdunstet ist, welcher der normalen bzw. durchschnittlichen Betriebszeit der beschriebenen Verdunstungseinrichtung entspricht. Somit kann der Benutzer der Verdunstungsvorrichtung ausschließlich durch Überprüfung des Behälters 55 bzw. des Flüssigkeitsniveaus im Behälter 55 erkennen, ob die Verdunstungsvorrichtung noch wirksam ist oder wie lange sie noch wirksam sein wird. Bei völlig entleertem Behälter 55 erkennt der Benutzer, daß das Trägerelement 3 zusammen mit seinen Behältern 55, 54 auszuwechseln und durch eine neue derartige Einheit zu ersetzen ist.

Das Ersetzen des Trägerelements 3 wird auf einfache Weise ermöglicht, indem die beiden Gehäuseteile 1,2 in der zur vorstehend beschriebenen Weise entgegengesetzten Richtung geöffnet werden, d.h. um die durch die Teile 48, 49, 19, 20, 21 festgelegte Achse voneinander weg geschwenkt werden.
Die oberen Kanten der beiden Gehäuseteile 1,2 werden hierzu in zueinander entgegengesetzter Richtung gezogen.
Nach dem Öffnen des Gehäuses läßt sich die Trägerplatte 3 entfernen. Die darin befindlichen Behälter 54, 55 sind vorteilhafterweise in die Trägerplatte 3 klemmend eingesetzt. Die Trägerplatte 3 wird durch eine neue, gefüllte Behälter 54, 55 aufweisende Trägerplatte ersetzt, welche mit ihrer unteren Kante 3a nach unten liegend so in die Gehäuseteile 1,2 eingesetzt wird, daß die untere Kante 3a innerhalb des wannenförmigen Körpers 17 zu liegen kommt und beide Behälterkörper 57, 60 in Richtung auf das Gehäuseteil 1 weisen.

Wie aus der Darstellung nach Fig. 6 hervorgeht, ist gemäß einer bevorzugten Ausführungsform ein begrenzter Öffnungswinkel zwischen den Gehäuseteilen 1 und 2 definiert. An dem Gehäuseteil 1 ist ein nasenförmiger Ansatz 31 vorgesehen, der mit dem unteren Teil des Schenkels 34, 35 bzw. einer senkrecht zur Gehäusewandung 36 verlaufenden Kurvenfläche 32 des Schenkels 34, 35 zusammenwirkt. Wie Fig. 2 und 6 zeigen, ist die kurvenförmige Bahn 32 der Schenkel 34,35 derart geformt, daß der bogenförmige Verlauf im Bereich der Bahn 32 in Richtung auf die Gehäusewand 36 in einen senkrecht zur Gehäusewand 36 verlaufenden Abschnitt 60 übergeht. Sobald dieser weitgehend gerade verlaufende Kantenabschnitt 60 in Berührung mit der Kante 31a gelangt und somit Führungskanten mit unterschiedlichem Krümmungsradius einander gegenüberstehen, wird zwischen den Gehäuseteilen 1,2 eine Sperrwirkung hervorgerufen, die einer weiteren Öffnungsbewegung zwischen den beiden Gehäuseteilen 1, 2 entgegenwirkt. Dadurch wird eine Weiterbewegung des Gehäuseteils 2 vom Gehäuseteil 1 weg um die Schwenkachse blockiert, solange nicht allzu hohe Kräfte auf die beiden Gehäuseteile 1, 2 auseinander ziehen.

Gemäß einer weiteren Ausführungsform der Verdunstungsvorrichtung ist das Gehäuseteil 2 derart konzipiert, daß an dem unteren, zur Gehäusewand 36 vorspringenden Teil der Schenkel 34,35 in der in Fig. 6 angedeuteten Weise Füße 64 ausgebildet sind, die durch parallel und vorzugsweise in der Ebene zur Gehäusewand 36 ausgebildete Filmscharniere 66 an dem Gehäuseteil 2 angelenkt sind. Demzufolge können die Füße 64 in die in Fig. 6 gezeigte Position, d.h. senkrecht zur Gehäusewand 36 liegend gestellt werden, um das gesamte Gehäuse auf einer planen Oberfläche abzustellen, oder sie können in die Ebene der Gehäusewand 36 hineingeklappt werden, so daß ein aus den Teilen 1 und 2 bestehendes Gehäuse an einer Wand befestigt oder angeklebt werden kann, wobei die Rückseite des Gehäuseteiles 2 an der Wand zur Anlage kommt.

Die vorstehend beschriebene Ausführungsform der Verdunstungsvorrichtung weist einen einzigen Behälter 54 für einen Wirkstoff auf. Anstelle eines einzigen Behälters können zwei oder mehr derartige Behälter benutzt werden mit einer entsprechenden Mehrzahl von Aufnahmeabschnitten 51 seitens des Gehäuseteils 1 und Öffnereinheiten an dem Gehäuseteil 2.

Gemäß einer weiteren Abwandlung der Erfindung ist vorgesehen, daß jeder Aufnahmeabschnitt 10 einen vorzugsweise vertikalen Schlitz enthält, der den dahinter befindlichen Wirkstoffbehälter 54 jedenfalls teilweise erkennen läßt und damit die Feststellung gestattet, ob nach Zusammenbau der beiden Gehäuseteile 1 und 2 in der Betriebsstellung eine Entleerung des Behälters 54 erfolgte oder nicht. Mittels dieses in der Zeichnung nicht gezeigten Schlitzes läßt sich ferner von außen feststellen, ob überhaupt ein Behälter 54 im Gehäuse vorhanden ist.
Bei der unter Bezugnahme auf Fig. 1 bis 6 beschriebenen Ausführungsform ist dagegen der Behälter 54 durch die Gehäusewand 6 vollständig abgedeckt, während der Behälter 55 mit seiner Indikatorflüssigkeit hinter der Öffnung 14 liegend von außen einsehbar angeordnet ist.

Die erfindungsgemäße Verdunstungsvorrichtung weist ein vorzugsweise aus nur zwei Gehäuseteilen bestehendes Gehäuse auf, die sich in einfacher Weise zusammensetzen lassen. Jedes Gehäuseteil ist einfach aufgebaut und kostengünstig herstellbar.
Die Öffnungsfunktion, die durch die Dorne 40,41 und 43 erzielt werden soll, läßt sich durch unterschiedliche Länge der Dorne erreichen. Bei der dargestellten Ausführungsform sind die den Behälter 54 öffnenden Dorne 40,41 von größerer Länge, um den Behälter 54 so stark zu öffnen, daß ein vollständiger kurzzeitiger Austritt der Wirkstofflüssigkeit und ein Ausfließen derselben in die durch den Körper 17 gebildete Wanne gewährleistet ist. Der Dorn 43 hat eine kürzere Länge und schmalere Spitze, um den Behälter 55 nur geringfügig zu perforieren, so daß Gegensatz zu einem plötzlichen oder kurzzeitigen Austritt der darin befindlichen Flüssigkeit eine langsame Verdunstung gesichert ist. Ersichtlicherweise ist die Größe des Behälters 55 und die Art der darin enthaltenen Flüssigkeit so abgestimmt, daß die darin enthaltene Flüssigkeit innerhalb eines Zeitraumes verdunstet, innerhalb welchem typischerweise die aus dem Behälter 54 ausgetretene Wirkstofflüssigkeit vollständig verbraucht bzw. verdunstet ist.

## Patentansprüche

1. Verdunstungsvorrichtung, insbesondere für flüchtige Wirkstoffe wie Insektizide, Duftstoffe oder dergleichen, mit einem Durchbrechungen aufweisenden Gehäuse, das aus wenigstens zwei um eine Schwenkachse beweglichen Gehäuseteilen besteht,
mit mindestens einem Aufnahmeabschnitt für einen Behälter für Wirkstoffe, und
mit mindestens einer dem Behälter zugeordneten Öffnereinheit
**dadurch gekennzeichnet,**
daß eine Einrichtung (14, 55) zur Anzeige des Betriebszustandes vorgesehen ist.

2. Verdunstungsvorrichtung, insbesondere für flüchtige Wirkstoffe wie Insektizide, Duftstoffe oder dergleichen,
mit einem Durchbrechungen aufweisenden Gehäuse, das aus wenigstens zwei um eine Schwenkachse beweglichen Gehäuseteilen besteht,
mit mindest einem Aufnahmeabschnitt für einen Behälter für Wirkstoffe, mit mindest einer dem Behälter zugeordneten Öffnereinheit,
**dadurch gekennzeichnet,**
daß eine Einrichtung (14, 55) zur Anzeige des Betriebszustandes vorgesehen ist, die aus einem Behälter (55) besteht, der eine Indikatorflüssigkeit enthält,
daß zur Lagerung des Behälters (55) mit Indikatorflüssigkeit ein Trägerelement (3) angeordnet ist, und
daß eines der Gehäuseteile (1, 2) eine Öffnung (14) enthält, hinter welcher der Behälter (55) mit der Indikatorflüssigkeit gehaltert ist.

3. Vorrichtung nach Anspruch 1,
**dadurch gekennzeichnet,**
daß die Einrichtung (14, 55) zur Anzeige des Betriebszustandes aus einem Behälter (55) besteht, der eine Indikatorflüssigkeit enthält.

4. Vorrichtung nach Anspruch 1 oder 3,
**dadurch gekennzeichnet,**
daß ein Trägerelement (3) zur Lagerung des Behälters (55) mit Indikatorflüssigkeit vorgesehen ist, und
daß eines der Gehäuseteile (1, 2) eine Öffnung (14) aufweist, hinter welcher der Behälter (55) mit der Indikatorflüssigkeit gehaltert ist.

5. Vorrichtung nach Anspruch 2 oder 4,
**dadurch gekennzeichnet,**
daß das Trägerelement (3) den Behälter (44) mit Wirkstoff lagert.

6. Vorrichtung nach wenigstens einem der vorangehenden Ansprüche,
**dadurch gekennzeichnet,**
daß das Trägerelement (3) wenigstens zwei Öffnungen (51, 52) aufweist.

7. Vorrichtung nach Anspruch 6,
**dadurch gekennzeichnet,**
daß die Öffnungen (51, 52) des Trägerelements (3) solche Form bzw. Größe haben, daß die Körper der Behälter (55 bzw. 54) fest und/oder klemmend aufgenommen werden.

8. Vorrichtung nach wenigstens einem der vorangehenden Ansprüche,
**dadurch gekennzeichnet,**
daß die Behälter (54, 55) eine plane, perforierbare Schicht aufweisen und daß jeder Behälter einen umlaufenden Rand oder Kragen (59, 61) aufweist, an welchem die perforierbare Schicht befestigt ist.

9. Vorrichtung nach wenigstens einem der vorangehenden Ansprüche,
**dadurch gekennzeichnet,**
daß zumindest einer der Behälter (54, 55) in eine Öffnung des Trägerelements (3) derart eingesetzt ist, daß der umlaufende Kragen (59, 61) auf der einen Seite und der Behälterkörper (57, 60) auf der anderen Seite des Trägerelements zu liegen kommt.

10. Vorrichtung nach wenigstens einem der vorangehenden Ansprüche,
**dadurch gekennzeichnet,**
daß eines der Gehäuseteile (1,2) an seiner unteren Kante einen wannenförmigen Körper (17) trägt.

11. Vorrichtung nach Anspruch 10,
**dadurch gekennzeichnet,**
daß der wannenförmige Körper (17) über ein Filmscharnier (16) schwenkfähig an einem der Gehäuseteile (1,2) gelagert ist.

12. Vorrichtung nach Anspruch 10 oder 11,
**dadurch gekennzeichnet,**
daß das den wannenförmigen Körper (17) tragende Gehäuseteil (1) von der zugehörigen Gehäusewand (6) abstehende Schenkel (7, 8) aufweist.

13. Vorrichtung nach Anspruch 12,
**dadurch gekennzeichnet,**
daß jeder Schenkel (7, 8) Öffnungen (19, 20) enthält und daß der wannenförmige Körper (17) entsprechend ausgebildete Vorsprünge (21) aufweist, die in den Öffnungen (19, 20) einrastbar sind.

14. Vorrichtung nach wenigstens einem der vorangehenden Ansprüche,
**dadurch gekennzeichnet,**
daß der in dem einen Gehäuseteil (1) ausgebildete Aufnahmeabschnitt (10), hinter welchem der Behälter (54) mit Wirkstoffflüssigkeit zu liegen kommt, in demjenigen Gehäuseteil vorgesehen ist, an welchem der wannenförmige Körper (17) ausgebildet ist.

15. Vorrichtung nach wenigstens einem der vorangehenden Ansprüche,
**dadurch gekennzeichnet,**
daß die Rastvorsprünge (21) des einen Gehäuseteils (1) als Verbindungselemente ausgebildet sind und zur Aufnahme von Zapfen (48) oder dergleichen angeordnet sind, die an dem anderen Gehäuseteil (2) vorgesehen sind.

16. Vorrichtung nach wenigstens einem der vorangehenden Ansprüche,
**dadurch gekennzeichnet,**
daß eine Einrichtung (31, 32, 49) zur Begrenzung der Relativbewegung der beiden Gehäuseteile (1,2) vorgesehen ist.

17. Vorrichtung nach wenigstens einem der vorangehenden Ansprüche,
**dadurch gekennzeichnet,**
daß jedes Gehäuseteil (1, 2) von der zugehörigen Gehäusewandung (6, 36) senkrecht abstehende Schenkel (7,8, 34, 35) aufweist.

18. Vorrichtung nach wenigstens einem der vorangehenden Ansprüche,
**dadurch gekennzeichnet,**
daß die Öffnereinheiten (40, 41, 43) durch Dorne mit gegebenenfalls unterschiedlicher Höhe und/oder Breite gebildet sind.

19. Vorrichtung nach wenigstens einem der vorangehenden Ansprüche,
**dadurch gekennzeichnet,**
daß an dem unteren Teil der Schenkel (7, 8, 34, 35) eines der Gehäuseteile (1, 2) umklappbare Füße (64) angebracht sind.
